**Europäisches Patentamt**

**European Patent Office**

**Office européen des brevets**

(19)

(11) Veröffentlichungsnummer: **0 233 327**

**A2**

(12)

# EUROPÄISCHE PATENTANMELDUNG

(21) Anmeldenummer: **86115518.2**

(22) Anmeldetag: **08.11.86**

(51) Int. Cl.⁴: **G01N 21/41**

Die Bezeichnung der Erfindung wurde geändert (Richtlinien für die Prüfung im EPA, A-III, 7.3).

(30) Priorität: **21.11.85 DE 3541154**

(43) Veröffentlichungstag der Anmeldung:
**26.08.87 Patentblatt 87/35**

(84) Benannte Vertragsstaaten:
**CH DE FR LI SE**

(71) Anmelder: **BROWN, BOVERI & CIE Aktiengesellschaft Kallstadter Strasse 1 D-6800 Mannheim 31(DE)**

(72) Erfinder: **Kurras, Erhard, Dipl.-Phys. Bahnhofstrasse 9-13 D-6900 Heidelberg(DE)**
Erfinder: **Meinhold, Henner, Dr. Dipl.-Phys. Zwischen den Bächen 14a D-6902 Sandhausen(DE)**
Erfinder: **Pistorius, Albert Rilkestrasse 13 D-6800 Mannheim 1(DE)**
Erfinder: **Weiser, Gerhard, Prof. Dr. Dipl.-Phys. Kellerwaldstrasse 2 D-3550 Marburg-Chappel(DE)**

(74) Vertreter: **Kempe, Wolfgang, Dr. et al c/o BROWN, BOVERI & CIE AG ZPT Postfach 351 D-6800 Mannheim 1(DE)**

(54) **Vorrichtung zum optischen Messen der Dichte von Gasen.**

(57) Die Erfindung bezieht sich auf eine Meßvorrichtung (1) zur Dichtebestimmung von Gasen in verschlossenen Kammern (2). Bei der erfindungsgemäßen Meßvorrichtung (1) wird von der Tatsache Gebrauch gemacht, daß der Brechungsindex von Gasen dichteabhängig ist. Die Meßvorrichtung (1) wird durch ein als Prisma (4) wirkendes Gehäuse gebildet, innerhalb dessen eine Gas enthalten ist, dessen Dichte der Dichte des Gases entspricht, das in die an die Meßvorrichtung (1) angeschlossene Kammer (2) eingefüllt ist. Das von einer Lichtquelle (15) kommende Licht wird durch das Gas in dem Prisma (4) hindurchgeleitet und an einer spiegelnden Wand (8) reflektiert. Mit einer Linse (10), die zuvor die von der Lichtquelle (15) kommenden Lichtstrahlen parallel ausgerichtet hat, werden die vom Prisma (4) reflektierten Lichtstrahlen auf einen Punkt fokussiert. In diesem Punkt ist das erste Ende eines Lichtleiters (12,13,14) angeordnet. Das auf einen Lichtleiter (12,13,14) auffallende Licht wird einer Auswerteeinrichtung (16) zugeführt, die dieses Lichtsignal in ein digitales Signal umwandelt und einer Anzeigevorrichtung zuführt.

Fig 1

## Meßvorrichtung

Die Erfindung bezieht sich auf eine Meßvorrichtung gemäß dem Oberbegriff des Patentanspruches 1.

Solche Meßvorrichtungen sind zur Dichtebestimmung von Gasen vorgesehen, die in geschlossene Gehäuse eingefüllt sind.

Schaltanlagen, die nach außenhin zu isolieren sind, werden oftmals druckgasisoliert ausgebildet. Die zur Isolierung vorgesehenen Kammern sind vorzugsweise mit reinem SF₆ gefüllt. Um Undichtigkeiten in den Kammern feststellen zu können, muß die Dichte des eingefüllten Gases des öfteren überprüft werden. Es ist deshalb erforderlich, daß jede gasgefüllte Kammer mit einer Meßvorrichtung ausgerüstet wird. Um die Kosten für solche Meßvorrichtungen in Grenzen zu halten, müssen diese relativ preisgünstig hergestellt werden können.

Der Erfindung liegt deshalb die Aufgabe zugrunde, eine Meßvorrichtung für geschlossene, gasgefüllte Einrichtungen zu schaffen, die einen einfachen Aufbau aufweist und leicht hergestellt werden kann.

Diese Aufgabe wird erfindungsgemäß durch die kennzeichnenden Merkmale des Patentanspruches 1 gelöst.

Die erfindungsgemäße Meßvorrichtung ist aus Bauelementen zusammengesetzt, die alle im Handel zu relativ niedrigen Preisen erhältlich sind. Hierdurch wird sichergestellt, daß die gesamte Meßvorrichtung sehr kostengünstig gefertigt werden kann. Die Meßvorrichtung ist für die Dichtebestimmung von Gasen beliebiger Art geeignet. Sie ist nicht auf die Messung von SF₆-Gas fixiert. Die Meßvorrichtung arbeitet nach einem optischen Prinzip und nutzt dabei die Tatsche aus, daß der Brechungsindex von Gasen dichteabhänig ist. Wird in eine gasdicht ausgebildete Kammmer nach ihrer Fertigstellung eine gewisse Menge Gas eingefüllt, so erfährt das von der Meßvorrichtung ausgesandte Licht hierdurch eine ganz bestimmte Brechung, die von der Dichte des Gases abhängig ist. Nach der Fertigstellung von Schaltanlagen werden deshalb anhand einer Meßreihe die für bestimmte Gasfüllungen spezifischen Brechungsindizes ermittelt, die dann bei der späteren Überprüfung der Kammern als Sollwerte verwendet werden. Wird beispielsweise die Kammer einer Schaltanlage für die Isolation mit einer bestimmten Menge an Gas gefüllt, so kann dieser eingefüllten Gasmenge ein bestimmter Brechungsindex zugeornet werden. Die Menge des eingefüllten Gases in der Kammer soll immer konstant bleiben, damit die Isolationswirkung der Schaltanlage erhalten bleibt. Ob eine Abweichung von dem Sollwert vorliegt, kann mit Hilfe der optisch arbeitenden Meßvorrichtung später jeder Zeit durch Überprüfen des Brechungsindex festgestellt werden. Da die Meßvorrichtung dauerhaft mit der Anlage verbunden wird, kann nach der Inbetriebnahme der Kammer kontinuierlich geprüft werden, ob der Brechungsindex des Gases noch dem Sollwert entspricht. Ist das nicht der Fall, so ist davon auszugehen, daß die Kammer eine Undichtigkeitsstelle aufweist. Erfindungsgemäß ist die Meßvorrichtung mit einem als Prisma wirkenden Gehäuse ausgestattet. Zwischen dem Innenraum dieses Gehäuses und der Kammer der Schaltanlage erfolgt ein Gasaustausch, und zwar derart, daß das im dem Prisma enthaltene Gas die gleiche Dichte aufweist. Der Gasaustausch erfolgt so genau, daß selbst geringste Dichteschwankungen in der Kammer auf den Innenraum des Prismas übertragen werden. Das Licht einer Lichtquelle wird durch das Prisma geleitet und dort reflektiert. Bleibt die Dichte des in dem Prisma enthaltenen Gases konstant, so wird das Licht immer unter dem gleichen Winkel reflektiert. Das reflektierte Licht wird einer Auswerteschaltung zugeführt. Diese ermittelt aus dem jeweiligen Reflexionswinkel, unter dem das Licht reflektiert wurde, die Dichte des Gases. Der ermittelte Wert wird angezeigt.

Weitere erfindungswesentliche Merkmale sind in den Unteransprüchen gekennzeichnet.

Die Erfindung wird nachfolgend anhand einer Zeichnung näher erläutert.

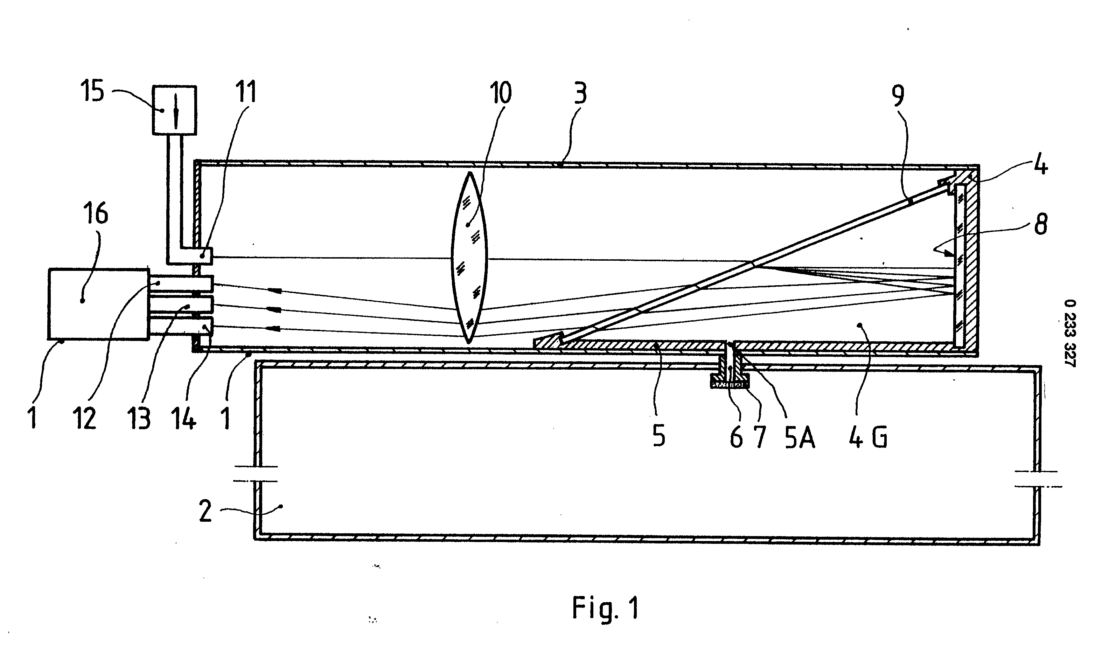

Figur 1 zeigt die erfindungsgemäße Meßvorrichtung 1, die an eine Kammer 2 einer nicht näher dargestellten Schaltanlage angeschlossen ist. Die Meßvorrichtung 1 weist als wesentliche Bauteile einen gasdichten Behälter 3, ein als Prisma wirkendes Gehäuse 4, eine Linse 10, vier Lichtleiter 11,12,13,14, eine Lichtquelle 15 und eine Auswerteschaltung 16 auf. Der Behälter 3 ist bei dem hier dargestellten Ausführungsbeispiel aus Metall gefertigt und auf der Kammer 2 angeordnet. Innerhalb des Behälters 3 ist das als Prisma 4 wirkende Gehäuse 4 installiert. Es ist gasdicht ausgebildet. Seine Grund platte 5 liegt auf dem Boden des Behälters 3 auf. Die Grundplatte 5 ist aus Metall oder einem Kunststoff gefertigt. Sie weist eine Öffnung 5A auf, die über eine Leitung 6 und ein Filter 7 den Innenraum des Prismas 4 mit dem Innenraum der Kammer 2 verbindet. Hierdurch wird ein Gesaustausch zwischen der Kammer 2 und dem Innenraum des Prismas erreicht. Selbst geringste Dichteschwankungen in dem Gas der Kammer 2 werden über die Leitung 6 auf den Innenraum des Prismas übertragen.

Das Gehäuse 4 weist einen Querschnitt in Form eines Dreiecks auf. Dieses wird durch die Grundplatte 5, eine auf der Innenseite spiegelnde Wand 8 und eine lichtdurchlässige Wand 9 begrenzt. Senkrecht zur Grundplatte 5 ist die auf der Innenseite spiegelnde Wand 8 angeordnet. Die dritte Wand 9, wird durch eine Glasplatte gebildet, die der Deckfläche des Behälters 3 zugewandt ist. Die Glasplatte 9 ist mit ihrem ersten Ende mit der Grundplatte 5 und mit ihrem zweiten Ende mit der spiegelnden Wand 8 gassdicht verbunden. Die Abmessungen der Grundplatte 5 und der Wand 8, insbesondere ihre Längen sind so bemessen, daß die Glasplatte 9 bei dem hier dargestellten Ausführungsbeispiel mit der Grundplatte 5 einen Winkel von etwa 10 Grad einschließt. Die übrigen Begrenzungswände (hier nicht dargestellt) des Gehäuses 4 sind aus einem gasdichten Material gefertigt, und mit den oben beschriebenen Wänden gasdicht verbunden. Vor dem Gehäuse 4, insbesondere vor seiner Glasplatte 9, ist der Lichtleiter 11 angeordnet, der mit der Lichtquelle 15 verbunden ist. Als Lichtquelle 15 dient bei dem hier dargestellten Ausführungsbeispiel eine Leuchtdiode. Die Austrittsöffnung des Lichtleiters 11 ist im Brennpunkt der als Sammellinse ausgebildeten Linse 10 angeordnet, die vorzugsweise eine Brennweite von etw 200mm aufweist. Diese richtet die von der Lichtquelle 15 kommmenden Strahlen parallel aus. Sie ist so angeordnet, daß alle von ihr kommenden Strahlen auf die Oberfläche der Glasplatte 9 geleitet werden. Innerhalb des Behälter 3 sind desweiteren die ersten Enden der Lichtleiter 11,12,13 und 14 angeordnet. Die übrigen Teile der Lichtleiter 11,12,13,14 sind aus dem Behälter 3 herausgeführt. Die zweiten Enden der Lichtleiter 12,13,14 sind an die Auswerteschaltung 16 angeschlosseen.

Die von der Linse 10 kommenden Lichtstrahlen treten durch die Glasplatte 9 hindurch und fallen auf die spiegelnde Wand 8 des Prismas 4. Dort werden sie reflektiert. Da die Anordnung Glasplatte 9 -gasgefüllter Innenraum des Gehäuses 4 -und spielgelnde Wand 8 die prismatische Wirkung des Gehäuses 4 hervorruft, werden die an der spiegelnden Wand 8 refektierten Strahlen unter einem Winkel, der von ihrem Einfallswinkel abweicht, wieder zu der Linse 10 zurückgeführt. Dieses von der Linse 10 wieder fokussierte Licht wird wegen des vom Einfallswinkel abweichenden Reflexionswinkels nicht mehr auf die Austrittsöffung des Lichtleiter 11 fokussiert. Viemehr liegt der Fokusierungspunkt in einem senkrechten Abstand dazu. Dieser Abstand wird durch die Dichte des in dem Gehäuse 4 enthaltenen Gases bestimmt.

Ist die Kammer 2 der Schaltanlage frei von Leckstellen, so weist das in ihr enthaltene Gas, bei dem hier beschriebenen Beispiel das SF₆ Gas, die gewünschte Dichte auf. Das gilt dann auch für das Gas in dem Prisma 4. Das von dort zur Linse 10 zurückkommnde Licht wird von dieser in einem Fokosierungspunkt abgebildet, der dem Sollwert entspricht. Der in diesem Fokosierungspunkt angeordnete Lichtleiter 12, insbesondere sein erstes Ende, wird von diesem Lichtbündel beleuchtet. Er leitet dieses als Meßsignal weiter zu der Auswerteschaltung 16. Dort wird das Lichtsignal in ein digitales Signal umgewandlet und einer Anzeigevorrichtung ( hier nicht dargestellt ) zugeführt. Nimmt die Dichte des in der Kammer 2 enthaltenen Gases ab, so sinkt auch die Dichte des Gases in dem Gehäuse 4. Dies bedeutet, daß das von der Lichtquelle 15 kommende Licht, insbesondere die parallelen Strahlen der Linse 10 innerhalb des Prismas 4 anders gebrochen werden und deshalb auch unter einem anderen Winkel wieder auf der Linse 10 auftreffen, als die Strahlen, die das Gas mit der gewünschten Dichte durchlaufen haben.

Dies bedeutet, daß das zurückgestrahlte Licht von der Linse 10 auf wieder einen anderen Punkt fokussiert wird. Dieser liegt wiederum in senkrechten Abstand zu dem ersten Fokosierungspunkt. Nach der Fertigstellung der Kammer 2 kann durch gezielte Dichteabnahme des Gases um jeweils 5 % der jeweils zugehörige Fokusierungspunkt ermittelt werden. In jedem dieser Fokusierungspunkte wird dann das Ende eines Lichtleiters 12,13,14 angeordnet. Das Ende des Lichtleiters 12 wird in dem Fokusierungspunkt angeordnet, der durch das Gas mit der gewünschten Dichte erzeugt wird, in dem sogenannten Sollpunkt. Die Enden der übrigen Lichtleiter 13,14 werden da positioniert, wo sich Fokusierungspunkte befinden, die durch ein Gas mit niedrigerer Dichte erzeugt werden.

Durch die Tatsache, daß der Brechungsindex von Gasen dichteabhängig ist, wird bewirkt, daß auch bei einem späteren Undichtwerden der Kammer 2 und der dadurch bedingten Dichteänderung des Gases die Fokusierungspunkte des von der Linse 10 kommenden Lichtes bei einer Dichteabnahme von 5 oder 10 % bzw. mehr immer wieder auf die Enden der Lichtleiter 13 und 14 abgebildet werden. Bei Bedarf kann die Meßvorrichtung auch mit mehr als drei Lichtleitern ausgestattet werden, die dann in entsprechenden Fokusierungspunkten angeornet werden. Die Aus werteschaltung 16 wertet das jeweils zugeführte Lichtsignal aus. Die ermittelte Dichte des Gases wird von einer Anzeigevorrichtung ( hier nicht dargestellt ), der das Ausgangssignal der Auswerteschaltung 16 zugeführt wird, angezeigt. Ist die Dichte des Gases unter einen kritschen Wert gefallen, so zeigt sie dies getrennt an und gibt Alarm, wenn die Schaltanlage abgsschaltet werden muß.

Um Wärmeeinwirkungen auf das Prisma 4 und damit nachteilige Einflüsse auf die Meßergebnisse auszuschalten, wird eine Linse 10 aus einem Material verwendet, das geeignet ist,Temperaturänderungen zu kompensieren. Eine Temperaturänderung bewirkt besonders eine Änderung in den Abmessungen der Grundplatte 5 des Prismas 4, wenn diese aus Metall gefertigt ist. Eine Kompensation dieser Änderung kann beispielsweise durch eine proportionale Änderung der Brennweite der verwendeten Linse 10 erzielt werden.
Luftdruckänderungen um etwa 20 % ergeben eine scheinbare $SF_6$-Dichteänderung von ca. 1 %. Um diesen Einfluß auszuschalten, wird der Behälter 3, innerhalb dessen das Prisma 4, die Linse 10 und die ersten Enden der Lichtleiter 11,12,13 und 14 angeordnet sind, mit Stickstoff ausgefüllt.

## Ansprüche

1. Meßvorrichtung zur Dichtebestimmung von Gasen in einer gasdicht geschlossenen Kammer - (2), insbesondere einer druckgasisolierten Schaltanlage, gekennzeichnet durch wenigstens ein als Prisma wirkendes Gehäuse (4), dessen Innenraum mit der Kammer (2) im Gasaustausch steht, und das mit wenigstens einer lichtdurchlässigen Wand - (9) versehen ist, vor der eine Lichtquelle (3) angeornet ist, deren an dem Gehäuse (4) reflektierte Strahlen als Meßsignale einer nachgeschalteten Auswerteschaltung (16) zugeführt sind.

2. Meßvorrichtung nach Anspruch 1, dadurch gekennzeichnet, daß das als Prisma (4) wirkende Gehäuse (4) einen Querschnitt in Form eines Dreiecks aufweist, das von einer Grundplatte (5), einer auf der Innenseite spiegelnden Wand (8) und der lichtdurchlässigen Wand (9) begrenzt ist.

3. Meßvorrichtung nach einem der Ansprüche 1 oder 2, dadurch gekennzeichnet, daß als Lichtquelle (15) eine Fotodiode vorgesehen ist, an die ein Lichtleiter (11) angeschlossen ist, der das Licht einer Linse (10) zuführt, die vor dem Prisma (4) angeordnet ist, und welche die von ihr parallel ausgerichteten Strahlen vollständig auf die lichtdurchlässige Wand (9) abbildet.

4. Meßvorrichtung nach einem der Ansprüche 1 bis 3, dadurch gekennzeichnet, daß die als Glasplatte ausgebildete lichtdurchlässige Wand (9) mit der Grundplatte (5) einen Winkel von etwa 10 Grad einschließt und auf der gegenüberliegenden Seite mit der auf der Innenseite spiegelnden Wand (8) ebenfalls gasdicht verbunden ist, und daß alle übrigen Wände des Gehäuses (4) aus einem gasdichten Material gefertigt und gasdicht miteinander verbunden sind.

5. Meßvorrichtung nach einem der Ansprüche 1 bis 4, dadurch gekennzeichnet, daß die Grundplatte (5) des Gehäuses (4) horzontal angeordnet und aus Metall oder Kunststoff gefertigt ist und mit der spiegelnden Wand(8) einen Winkel von 90 Grad einschließt.

6. Meßvorrichtung nach einem der Ansprüche 1 bis 5, dadurch gekennzeichnet, daß der Innenraum des Gehäuses (4) mit dem Gas gleicher Dichte ausgefüllt ist, das auch in der Kammer (2) enthalten ist, und daß für den Gasaustausch mit der Kammer (2) eine Öffnung (5A) in der Grundplatte (5) des Prismas (4) vorgesehen ist, von der eine Leitung (6) mit eingebautem Filter (7) zu dem Innenraum der Kammer (2) geführt ist.

7. Meßvorrichtung nach einem der Ansprüche 1 bis 6, dadurch gekennzeichnet, daß eine Vielzahl weiterer Lichtleiter (12,13,14) vorgesehen sind, und daß die Linse (10) so angeordnet ist, daß die vom Prisma (4) reflektierten Strahlen auf das Ende wenigstens eines dieser Lichtleiter (12,13,14) fokussiert ist

8. Meßvorrichtung nach einem der Ansprüche 1 bis 7, dadurch gekennzeichnet, daß das Prisma - (4), die Linse (10) und die ersten Enden der Lichtleiter (11,12,13,14) innerhalb eines gasdichten Behälters (3) angeordnet sind, der mit Stickstoff gefüllt ist.

9. Meßvorrichtung nach einem der Ansprüche 1 bis 8, dadurch gekennzeichnet, daß zum Ausgleich von Temperaturänderungen eine Linse (10) vorgesehen ist, deren Brennweite sich an die Temperaturänderungen anpaßt.

Fig. 1